# EUROPEAN PATENT SPECIFICATION

(11) **EP 1 183 022 B1**
(45) Date of publication and mention of the grant of the patent: **29.12.2010**
(21) Application number: 00925997.9
(22) Date of filing: 14.04.2000
(51) Int. Cl.: A61K 31/19, A61K 31/415

(54) **METHOD AND COMPOSITION FOR TREATING MAMMALIAN NASAL AND SINUS DISEASES CAUSED BY INFLAMMATORY RESPONSE**
VERFAHREN UND ZUSAMMENSETZUNG ZUR BEHANDLUNG VON ENTZÜNDUNGSREAKTION BEDINGTEN NASEN- UND NEBENHÖLENKRANKHEITEN IN SÄUGETIEREN
METHODE ET COMPOSITION PERMETTANT DE TRAITER LES MALADIES DU NEZ ET DES SINUS PROVOQUEES PAR UNE REACTION INFLAMMATOIRE CHEZ LES MAMMIFERES

(30) Priority: 14.05.1999 US 312168
(43) Date of publication of application: 06.03.2002
(73) Proprietor: Cellular Sciences, Inc., Flemington, NJ 08822 (US)
(72) Inventor: KATZ, Stanley, E., Milltown, NJ 08850 (US); MARTIN, Alain, Ringoes, NJ (US)
(74) Representative: Webber, Philip Michael
(86) International application number: PCT/US2000/010062
(87) International publication number: WO 2000/069431

(56) References cited:
- WO-A-95/13810
- US-A- 5 240 694
- US-A- 5 478 565

## Description

### BACKGROUND OF THE INVENTION

### 1. Field of the Invention

This invention pertains to compositions for preventing and treating the damage and resulting disease state in mammals caused by mammalian nasal and sinus cells involved in the inflammatory response resulting in undesired respiratory bursting, production of enzymes and cellular signaling agents in mammalian cells.

### Description of the Prior Art

Reactive oxygen species are generated by cells in response to inter alia aerobic metabolism, catabolism of drugs, toxins and other xenobiotics, ultraviolet and x-ray radiation and the respiratory burst of phagocytic cells (such as white blood cells) to kill invading bacteria and in response to foreign bodies. Hydrogen peroxide, for example, is produced during respiration of most living organisms especially by stressed and living cells.

These active oxygen species can injure cells. An important example of such damage is lipid peroxidation which involves the oxidative degradation of unsaturated lipids. Lipid peroxidation is highly detrimental to membrane structure and function and can cause numerous cytopathological effects. Cells defend against lipid peroxidation by producing radical scavengers such as superoxide dismutase, catalase, and peroxidase. Injured cells have a decreased ability to produce radical scavengers. Excess hydrogen peroxide can react with pyrimidines to open the 5, 6-double bond. This reaction inhibits the ability of pyrimidines to hydrogen bond to complementary bases, Hallaender et al. (1971). Such oxidative biochemical injury can result in the loss of cellular membrane integrity, reduced enzyme activity, changes in transport kinetics, changes in membrane lipid content, and leakage of potassium ions, amino acids, and other cellular material.

The production of reactive oxygen intermediates has been suggested to cause many skin, tissue, and organ disorders such as atherosclerosis, arthritis, cytotoxicity, skin inflammation, photoaging, wrinkling, actinic keratosis, tumor formation, cancer, hypertension, Parkinson's disease, lung disease, and heart disease. The role of active oxygen radicals in promoting tumors has been proposed based on the findings that (a) tumor promoters increase the level of oxygen radicals, (b) many free radical generating systems promote tumors, and (c) certain antioxidants inhibit the biochemical effects of tumor promoters.

In Vitro, reactive oxygen intermediates can be generated in cellular culture media by autooxidation and photooxidation of media components. During excision and storage, transplant organs can suffer oxidative injuries which result in thee loss of cellular membrane integrity and shorten the usable life of the organ.

When cells are stressed by oxidative injury, a resuscitation step is necessary to recondition the cells. Antioxidants have been shown to inhibit damage associated with active oxygen species. For example, pyruvate and other alphaketoacids have been reported to react rapidly and stoichiometrically with hydrogen peroxide to protect cells from cytolytic effects, O'Donnell-Tormey et al., J. Exp. Med., 165, pp. 500-514 (1987).

United States Patent No. 5,798,388 issued to Katz discloses the treatment of airway diseases of the lungs such as bronchial asthma, acute bronchitis, emphysema, chronic obstructive emphysema, centrilobular emphysema, panacinar emphysema, chronic obstructive bronchitis, reactive airway disease, cystic fibrosis, bronchiectasis, acquired bronchiectasis, kartaagener's syndrone, atelectasis, acute atelectasis, chronic atelectasis, pneumonia, essential thrombocytopenia, legionnaires disease, psittacosis, fibrogenic dust disease, diseases due to organic dust, diseases due to irritant gases and chemicals, hypersensitivity diseases of the lung, idiopathic infiltrative diseases of the lungs and the like by inhaling pyruvate containing compositions. The pyruvate acts as a inflammatory response mediator and reduces the undesired response in the lungs.

United States Patent No. 5,210,098 issued to Nath discloses a method to arrest or prevent acute kidney failure by administration of a non-toxic pyruvate salt to a patient in need of such treatment.

The Nath invention provides a therapeutic method comprising administration of an amount of pyruvate salt to a patient experiencing, or in danger of, acute renal failure. The pyruvate salt, preferably sodium pyruvate, is preferably dispersed or dissolved in a pharmaceutically acceptable liquid carrier and administered parenterally in an amount effective to arrest or prevent said acute renal failure, thus permitting restoration of normal kidney function. In some cases, the pyruvate may be infused directed into the kidney or into the proximal renal arterial circulation. The method is effective to prevent or counteract acute kidney failure due to a wide variety of causes, including, but not limited to, traumatic injury, including burn injury and obstruction; reperfusion following ischemia, inflammatory glomerulonephritis, and sepis, e.g. due to gram negative bacterial infection.

Martin et al., 1994, United States patent no. 5,296,370, discloses therapeutic compositions for preventing and reducing injury to mammalian cells and increasing the resuscitation rate of injured mammalian cells. In one embodiment, the therapeutic composition comprises (a) pyruvate selected from the group consisting of pyruvic acid, pharmaceutically acceptable salts of pyruvic acid, and mixtures thereof, (b) an antioxidant, and (c) a mixture of saturated and unsaturated fatty acids wherein the fatty acids are those fatty acids required for the resuscitation of injured mammalian cells.

United States Patent No. 5,256,697 issued to Miller et al., discloses a method for orally administering a therapeutically effective amount of pyruvate precursor to a mammal to improve insulin resistance, lower lasting insulin levels and reduce fat gain.

United States patent nos. 3,920,835, 3,984,556, and 3,988,470, all issued to Van Scott et al. disclose methods for treating acne, dandruff, and palmar keratosis, respectively, which consist of applying to the affected area a topical composition comprising from about 1% to about 20% of a lower aliphatic compound containing from two to six carbon atoms selected from the group consisting of alpha-hydroxyacids, alpha-ketoacids and esters thereof, and 3-hydroxybutryic acid in a pharmaceutically acceptable carrier. The aliphatic compounds include pyruvic acid and lactic acid.

United States patents nos. 4,105,783 and 4,197,316, both issued to Yu et al., disclose a method and composition, respectively, for treating dry skin which consists of applying to the affected area a topical composition comprising from about 1% to about 20% of a compound selected from the group consisting of amides and ammonium salts of alpha-hydroxyacids, beta-hydroxyacids, and alpha-ketoacids in a pharmaceutically acceptable carrier. The compounds include the amides and ammonium salts of pyruvic acid and lactic acid.

United States patent no. 4,234,599, issued to Van Scott et al., discloses a method for treating actinic and nonactinic skin keratoses which consists of applying to the affected area a topical composition comprising an effective amount of a compound selected from the group consisting of alpha-hydroxyacids, beta-hydroxyacids, and alpha-ketoacids in a pharmaceutically acceptable carrier. The acidic compounds include pyruvic acid and lactic acid.

United States patent no. 4,294,852, issued to Wildnauer et al., discloses a composition for treating skin which comprises the alpha-hydroxyacids, beta-hydroxyacids, and alpha-ketoacids disclosed above by Van Scott et al. in combination with C3-C8 aliphatic alcohols.

United States patent no. 4,663,166, issued to Veech, discloses an electrolyte solution which comprises a mixture of L-lactate and pyruvate in a ratio from 20:1 to 1:1, respectively, or a mixture of D-beta-hydroxybutyrate and acetoacetate, in a ratio from 6:1 1 to 0.5:1, respectively.

Sodium pyruvate has been reported to reduce the number of erosions, ulcers, and hemorrhages on the gastric mucosa in guinea pigs and rats caused by acetylsalicylic acid. The analgesic and antipyretic properties of acetylsalicylic acid were not impaired by sodium pyruvate, Puschmann, Arzneimittelforschung, 33, pp. 410-415 and 415-416 (1983).

Pyruvate has been reported to exert a positive inotropic effect in stunned myocardium, which is a prolonged ventricular dysfunction following brief periods of coronary artery occlusions which does not produce irreversible damage, Mentzer et al., Ann.Surg., 209, pp. 629-633 (1989).

Pyruvate has been reported to produce a relative stabilization of left ventricular pressure and work parameter and to reduce the size of infarctions. Pyruvate improves resumption of spontaneous beating of the heart and restoration of normal rates and pressure development, Bunger et al., J. Mol. Cell. Cardiol., 18, pp. 423-438 (1986), Mochizuki et al., J. Physiol. (Paris), 76, pp. 805-812 (1980), Regitz et al., Cardiovasc, Res., 15 pp. 652-658 (1981), Giannelli et al., Ann.Thorac. Surg., 21 pp. 386-396. (1976).

Sodium pyruvate has been reported to act as an antagonist to cyanide intoxication (presumably through the formation of cyanohydrin) and to protect against the lethal effects of sodium sulfide and to retard the onset and development of functional, morphological, and biochemical measures of acrylamide neuropathy of axons, Schwartz et al., Toxicol. Appl. Pharmacol., 50 pp. 437-442 (1979), Sabri et al., Brain Res., 483, pp. 1-11 (1989).

A chemotherapeutic cure of advanced L1210 leukemia has been reported using sodium pyruvate to restore abnormally deformed red blood cells to normal. The deformed red blood cells prevented adequate drug delivery to tumor cells, Cohen, Cancer Chemother, Pharmacol., 5, pp. 175-179 (1981).

Primary cultures of heterotopic tracheal transplant exposed in vivo to 7, 12-dimethylbenz(a)anthracene were reported to be successfully maintained in enrichment medium supplemented with sodium pyruvate along with cultures of interleukin-2 stimulated peripheral blood lymphocytes, and plasmacytomas and hybridomas, pig embryos, and human blastocysts, Shacter, J. Immunol, Methods, 99, pp. 259-270 (1987), Marchok et al., Cancer Res., 37, pp. 1811-1821 (1977), Davis, J. Reprod. Fertil, Suppl., 33. pp 115-124 (1985), Okamoto et al., No To Shinkei, 38, pp. 593-598 (1986), Cohen et al., J. In Vitro Fert. Embryo Transfer, 2, pp. 59-64 (1985).

United States patents nos. 4,158,057, 4,351,835, 4,415,576, and 4,645,764, all issued to Stanko, disclose methods for preventing the accumulation of fat in the liver of a mammal due to the ingestion of alcohol, for controlling weight in a mammal, for inhibiting body fat while increasing protein concentration in a mammal, and for controlling the deposition of body fat in a living being, respectively. The methods comprise administering to the mammal a therapeutic mixture of pyruvate and dihydroxyacetone, and optionally riboflavin. United States patent no. 4,548,937, issued to Stanko, discloses a method for controlling the weight gain of a mammal which comprises administering to the mammal a therapeutically effective amount of pyruvate, and optionally riboflavin. United States patent no 4,812,479, issued to Stanko, discloses a method for controlling the weight gain of a mammal which comprises administering to the mammal a therapeutically effective amount of dihydroxyacetone, and optionally riboflavin and pyruvate.

Rats fed a calcium-oxalate lithogenic diet including sodium pyruvate were reported to develop fewer urinary calculi (stones) than control rats not given sodium pyruvate, Ogawa et al., Hinvokika Kivo, 32, pp. 1341-1347 (1986).

United States patent no. 4,521,375, issued to Houlsby, discloses a method for sterilizing surfaces which come into contact with living tissue. The method comprises sterilizing the surface with aqueous hydrogen peroxide and then neutralizing the surface with pyruvic acid.

United States patent no. 4,416,982, issued to Tauda et al., discloses a method for decomposing hydrogen peroxide by reacting the hydrogen peroxide with a phenol or aniline derivative in the presence of peroxidase.

United States patent no, 4,696,917, issued to Lindstrom et al., discloses an irrigation solution which comprises Eagle's Minimum Essential Medium with Earle's salts, chondroitin sulfate, a buffer solution, 2-mercaptoethanol, and a pyruvate. The irrigation solution may optionally contain ascorbic acid and alpha-tocopherol. United States patent no. 4,725,586, issued to Lindstrom et al., discloses an irrigation solution which comprises a balanced salt solution, chondroitin sulfate, a buffer solution, 2 mercaptoethanol, sodium bicarbonate or dextrose, a pyruvate, a sodium phosphate buffer system, and cystine. The irrigation solution may optionally contain ascorbic acid and gamma-tocopherol.

United States patent no. 4,847,069, issued to Bissett et al., discloses a photoprotective composition comprising (a) a sorbohydroxamic acid, (b) an anti-inflammatory agent selected from steroidal anti-inflammatory agents and a natural anti-inflammatory agent, and (c) a topical carrier. Fatty acids may be present as an emollient. United States patent no. 4,847,071, issued to Bissett et al., discloses a photoprotective composition comprising (a) a tocopherol or tocopherol ester radical scavenger, (b) an anti-inflammatory agent selected from steroidal anti-inflammatory agents and a natural anti-inflammatory agent, and (c) a topical carrier. United States patent no. 4,847,072, issued to Bissett et al., discloses a topical composition comprising not more than 25% tocopherol sorbate in a topical carrier.

The addition of sodium pyruvate to bacterial and yeast systems has been reported to inhibit hydrogen peroxide production, enhance growth, and protect the systems against the toxicity of reactive oxygen intermediates. The optimum ratio of unsaturated to saturated fatty acids contained within chicken fat enhanced membrane repair and reduced cytotoxicity. The antioxidants glutathione and thioglycollate reduced the injury induced by oxygen radical species, Martin, Ph.D. thesis, (1987 -89).

While the above therapeutic compositions and methods are reported to inhibit the production of reactive oxygen intermediates, none of the compositions and methods treats the damage and resulting disease state in mammals caused by undesired respiratory bursting, production of enzymes and cellular signaling agents in mammalian nasal and sinus cells.

Allergic reactions include four types of reactions, i.e., types I, II, III and IV. The type I (immediate-type, anaphylactic) allergic reaction is triggered by the reaction-relating-factor immunoglobulin E (hereinafter abbreviated as an IgE antibody). The reaction steps can be divided roughly into the following three steps. The first step is a sensitization step involving IgE antibody production and binding of the resulting IgE antibodies to mast cells or basophils. The second step involves degranulation of the mast cells or basophils and release of chemical mediators. The third steps involves onset of effects of the released chemical mediators on the target organs. Thus, the type I allergic reaction against foreign antigens leads to onset of symptoms through the above reaction steps.

Only symptomatic treatments by inhibiting the above second and/or third reaction steps have been carried out to treat allergic diseases. That is, the treatments are carried out by inhibiting the release of chemical mediators accompanying the degranulation and/or by inhibiting allergic reactions induced by the released chemical mediators. These symptomatic treatments have been known to be effective not only in systemic administration of anti-allergic agents but also in their topical administration to the nose, etc. However, the effects of the treatments are limited because the treatments do not inhibit IgE antibody production which is the basic first step of the type I allergic reaction.

However, because the mechanisms of nasal topical IgE antibody production are not clear, there is no report on effects of nasally topically administered drugs applicable to nasal topical membrane allergic reaction. As described above, there is no satisfactory anti-allergic pharmaceutical compositions that are effective and safe in nasal topical administration.

The present invention provides an excellent anti-allergic pharmaceutical composition for nasal topical use.

### SUMMARY OF THE INVENTION

The present invention pertains to compositions for treating the disease state in mammals caused by mammalian nasal and sinus cells involved in the inflammatory response as claimed herein. The method for treating the disease state in mammals caused by mammalian nasal and sinus cells involved in the inflammatory response preferably comprises: contacting cells of the mucous membranes of nasal and sinus passages of a mammalian subject with an amount of the inflammatory mediator which is capable of reducing an undesired inflammatory response in said cells.

The inflammatory mediator in addition to reducing the undesired inflammatory response and being an antioxidant, may further provide a cellular energy source and be a building block in the cellular synthesis of other cellular components. The inflammatory mediator may also increase cellular metabolic rate.

The present invention also pertains to compositions for reducing and treating the disease state in mammals caused by undesired inflammatory response of nasal and sinus cells as claimed herein.

The inflammatory response mediators may be used individually, in combination and further in combination with a therapeutic agent such as an antibacterial, antiviral, antifungal, protein, enzyme, antihistamine, hormone, nonsteroidal anti-inflammatory, cytokine, vitamin (vitamin C and vitamin E), insulin and steroid.

A preferred method of administering the inflammatory mediator is by oral and/or nasal inhalation and nose drops.

### DETAILED DESCRIPTION OF THE INVENTION

Therapeutic compositions and a method for treating the disease state in mammals caused by mammalian nasal and sinus cells involved in the inflammatory response have been discovered. The mammalian cells primarily responsible for the inflammatory response are white blood cells or leucocytes.

In a method for treating the disease state in mammals caused by mammalian cells involved in the inflammatory response, mammalian nasal and sinus cells are contacted with an inflammatory mediator. The inflammatory mediator is present in an amount capable of reducing the undesired inflammatory response and is an antioxidant.

The inflammatory response, often referred to as respiratory bursting, is the response of defensive mammalian cells primarily white blood cells or leucocytes. These cells normally respond to an injury or invasion of the mammal by releasing a number of active compounds at the injury or invasion site. Among the compounds released are enzymes such as proteases, histamine and active oxygen species such as hydrogen peroxide.

A purpose of the respiratory burst is to provide a battery of oxidizing agents in response to a stimulant that can be used by the leucocytes for the destruction of foreign cells, viruses, particulates and some toxins which have been ingested by or are in the vicinity of the leucocyte. The term "respiratory burst" refers to a coordinated series of metabolic events that take place when leucocytes are exposed to appropriate stimuli. This group of events underlies all oxygen dependent killings by leucocytes.

The first of these events is the sharp increase in oxygen uptake that occurs upon stimulation of the leucocytes. While oxygen consumption by resting leucocytes varies widely by cell type, all respond to appropriate stimuli with an increase in oxygen uptake.

Stimulation of the leucocyte also causes an increase in glucose oxidation via the hexose monophosphate shunt. The hexose monophosphate shunt is a metabolic pathway in which glucose is oxidized to carbon dioxide and a five carbon sugar, with NADP+ serving as electron acceptor. Activation of the hexose monophosphate shunt therefore means that the oxidation of NADPH to NADP+ increases during the respiratory burst.

The respiratory burst produces superoxide and hydrogen peroxide. Oxygen taken up by the respiratory burst is converted to superoxide. Hydrogen peroxide appears to arise during the respiratory burst mainly from the dismutation of superoxide anion.

O-₂ + O-₂ + 2H⁺ = 2 H₂ O₂ + O₂

It has been demonstrated by Root and Metcalf and reported in J. Clin. Invest. 60:1266 that 80 percent of the superoxide is converted to hydrogen peroxide, and this dismutation reaction is the only important source of the hydrogen peroxide generated during the burst. Hydrogen peroxide and superoxide are believed to be responsible for the killing by leucocytes.

Many agents, both soluble and particulate, are able to activate the respiratory burst. Particulate activating agents include bacteria, viruses and fungi for internal body organs or areas and bacteria, viruses, fungi, fibers, smoke, dust, ash, pollen, smog and the like for body cavities and organs such as the lungs, skin, digestive and excretory tracks open to the environment. Soluble agents can be toxins, medicinal compounds and soluble excretions of bacteria, fungi and infected mammalian cells and the like.

Activation of the respiratory burst in leucocytes usually follows exposure to the stimulus for less than a minute. Upon stimulation of the respiratory burst, the consumption of oxygen in leucocytes increases by over 100 fold resulting in, among other things, the production of superoxide, peroxide and hydrogen peroxide. The term "leucocytes" as used herein includes lymphocytes, phagocytes, macrophages and auxiliary cells.

Usually, after respiratory bursting the stimulant and/or the mechanism of stimulation turns off allowing the leucocyte to return to its normal resting state. When the bursting does not turn off, the inflammatory action of the leucocytes continues unchecked causing a number of disease states. These disease states occur as the compounds produced by the leucocytes attack, injure and kill tissue cells and other leucocytes. It is this failure to turn off the respiratory burst and the resulting injury to surrounding tissue cells, blood cells, other leucocytes and injured cells that produces the disease states treated by the present invention. Undesired inflammatory response occurs when the inflammatory response causes injury to host cells and this injury poses an independent threat to the host.

In a preferred embodiment, the therapeutic compositions containing an inflammatory mediator are administered locally to the site of inflammation. In another preferred embodiment, the therapeutic compositions are administered systemically. In yet another preferred embodiment, the therapeutic compositions are administered systemically and locally concomitantly.

In a preferred embodiment, the therapeutic compositions are administered by nasal inhalation. In another preferred embodiment, the therapeutic compositions are administered by nose drops. The therapeutic compositions may be first nebulized by any suitable means. The therapeutic compositions may be in liquid or solid form with liquid droplets or particle size being small enough to facilitate access to nasal and sinus tissue by inhalation or nose drops.

In another preferred embodiment, a sterile solution of therapeutic agent is nebulized and inhaled by the patient. A therapeutically effective amount of inflammatory medication is inhaled. This may be accomplished in a single inhalation or by repeated inhalations over a period of time typically 1 to 30 minutes. Preferably, inhalation will be complete in one or two inhalations or applications of nose drops.

The term "injured cell" as used herein means a cell which has some or all of the following: (a) injured membranes so that transport through the membranes is diminished and may result in one or more of the following, an increase in toxins and normal cellular wastes inside the cell and/or a decrease in nutrients and other components necessary for cellular repair inside the cell, (b) an increase in concentration of oxygen radicals inside the cell because of the decreased ability of the cell to produce antioxidants and enzymes, and (c) damaged DNA, RNA and ribosomes which must be repaired or replaced before normal cellular functions can be resumed.

Preferably the inflammatory mediator when brought into contact with a mammalian nasal and sinus cell provides a cellular energy source and a building block in the cellular synthesis of other cellular components.

The inflammatory response being reduced is preferably at least one of the following: oxygen radical production, peroxide production, cytokine and/or protease production, prostiglandin production, erythema, histamine and interlukin production and like responses known in the art as inflammatory responses.

The inflammatory mediator of the invention is at least one compound selected from the group consisting of a pyruvate precursor, pyruvate or a mixture thereof. A precursor is a substance from which another substance is formed and in this text also includes salts.

Preferably the pyruvate is selected from the group consisting of pyruvic acid, lithium pyruvate, sodium pyruvate, potassium pyruvate, magnesium pyruvate, calcium pyruvate, zinc pyruvate, manganese pyruvate, and the like and mixtures thereof. Sodium pyruvate is most preferred.

The pyruvate precursor of the invention is selected from the group consisting of pyruvyl-glycine, pyruvyl-alanine, pyruvyl-cysteine, pyruvyl-leucine, pyruvyl-valine, pyruvyl-isoleucine, pyruvyl-phenylalanine, pyruvamide, dihydroxyacetone, and salts of pyruvic acid.

Preferred salts of the inflammation mediator are salts that do not produce an adverse effect on the mammalian cell when applied as a salt of the inflammation mediator. Typical salts would be the lithium, sodium, potassium, aluminum, magnesium, calcium, zinc, manganese, ammonium and the like and mixtures thereof.

Compositions for reducing and treating the disease state in mammals caused by undesired inflammatory response comprise: an inflammatory response mediator; and a carrier composition.

The carrier composition is preferably selected from the group consisting of tablets, capsules, liquids, isotonic liquids, isotonic media, enteric tablets and capsules, parenterals, topicals, creams, gels, ointments, chewing gums, confections and the like.

The inflammatory mediator is preferably administered in a therapeutically effective amount to reduce the undesired inflammatory response, preferably from 0.0001 to 10 grams per dose, and more preferably 0.0001 to 1 gram per dose and most preferably 0.001 to 0.25 grams per dose. It is understood that the method of administration and the condition being treated will greatly affect the dose required to achieve the therapeutic effect.

The inflammatory mediator of the present invention is for treating rhinitis, eosinophilia syndrome and/or sinusitis. The present invention also discloses a pyruvate containing nasal moisturizing saline solution as claimed herein. This may be used for the prevention and/or treatment of rhinitis, eosiophilia syndrome, sinusitis and related conditions associated with nasal congestion.

In general, the nasal moisturizing saline solution is comprised of pure water; sodium chloride, 0.65% by weight; pyruvate, at least 0.1mM; buffer; and optionally a preservative, where the nasal solution is buffered and made isotonic. The pyruvate is preferably present in the nasal solution at a concentration range of between from about 0.1mM to about 10mM. The buffer is preferably used to maintain physiological pH. Any buffer or buffer system which is capable of maintaining physiological pH may be used. Examples of acceptable buffers and buffer systems include sodium bicarbonate, disodium phosphate/sodium phosphate, and monobasic potassium phosphate/sodium hydroxide. Likewise, any antiseptic preservative which is capable of preserving the sterility of the nasal moisturizing saline solution may be used. Examples of acceptable preservatives include phenylcarbinol, benzalkonium chloride, and thimerosal.

In a preferred embodiment, the pyruvate is present in the nasal moisturizing saline solution at a concentration of about 0.5mM to 10mM, the buffer is sodium bicarbonate.

In another preferred embodiment, the pyruvate is present in the nasal solution at a concentration of about 0.5mM to 6mM, the buffer is sodium bicarbonate.

In yet another preferred embodiment, the pyruvate is present in the nasal solution at a concentration of about 1.0mM to 6mM, the buffer is sodium bicarbonate.

Since this invention discloses a solution that is administered to the mucous membranes of the nasal and sinus passages of a human, it is important to maintain sterile conditions throughout the preparation of the nasal moisturizing saline solution. The following specific Examples are used to illustrate the pyruvate-containing nasal solution of the present invention.

### EXAMPLE 1 NASAL SOLUTION

1.875 fl. oz. (55 ml) of nasal saline solution may be purchased commercially over the counter (Phar-Mor, Inc., Youngstown, Ohio). The solution containes purified water, sodium chloride (0.65% by weight), is buffered and made isotonic with sodium bicarbonate, and contains phenylcarbinol as a preservative.

A 500mM pyruvate solution in water may be prepared. The solution contains purified water, and 500mM pyruvate.

0.3ml of the 500mM pyruvate solution is added to 1.0 fl. oz. (30 ml) of nasal saline solution, thereby yielding a nasal saline solution containing approximately 5mM pyruvate.

### EXAMPLE 2 NASAL SOLUTION

1.5 fl. oz. (44 ml) of nasal saline solution may be purchased commercially over the counter (Perrigo.RTM., Allegan, Mich.). The solution contains purified water, sodium chloride (0.65% by weight), is buffered and made isotonic with sodium bicarbonate, and contains phenylcarbinol as a preservative.

A 500mM pyruvate solution in water may be prepared. The solution contained purified water, and 500mM pyruvate.
0.3ml of the 500mM pyruvate solution is added to 1.0 fl. oz. (30 ml) of nasal saline solution, thereby yielding a nasal saline solution containing approximately 5mM pyruvate.

### EXAMPLE 3 NASAL SOLUTION

1.5 fl. oz. (45 ml) of Afrin.RTM. moisturizing saline mist solution may be purchased commercially over the counter (Schering-Plough, Memphis, Tenn.). The solution contains water, PEG-32, sodium chloride, PVP, disodium phosphate, sodium phosphate, benzalkonium chloride, and disodium EDTA.

A 500mM pyruvate solution in water may be prepared. The solution contained purified water, and 500mM pyruvate.

0.3ml of the 500mM pyruvate solution is added to 1.0 fl. oz. (30 ml) of nasal saline solution, thereby yielding a nasal saline solution containing approximately 5mM pyruvate.

### EXAMPLE 4 THERAPEUTIC NASAL SOLUTION

0.5 fl. oz. (15 ml) of DristanØ nasal spray solution was purchased commercially over the counter. The solution contained Oxymetazoline HCl 0.05% by weight, benzalkonium chloride 1:5000 in a buffered isotonic aqueous solution, hydroxypropylmethylcellulose, potassium phosphate, sodium chloride, sodium phosphate, thimerosal preservative 0.002% and water.

A 500mM pyruvate solution in water may be prepared. The solution contained purified water, and 500mM pyruvate.

0.3ml of the 500mM pyruvate solution is added to 1.0 fl. oz. (30 ml) of DristanØ nasal spray solution, thereby yielding a 0.05% oxymetazoline HCl solution containing approximately 5mM pyruvate.

### Administration

In the use for the prevention and/or treatment of rhinitis, eosiophilia syndrome, sinusitis and related conditions associated with nasal congestion, the pyruvate-containing nasal solution is administered to the nostrils of a patient in need thereof. The nasal moisturizing saline solution is comprised of water; sodium chloride, 0.65%; 0.1 to 6mM pyruvate; at least 0.001% by weight buffer; and a preservative, where the nasal solution is buffered and made isotonic.

The pyruvate is present in the nasal moisturizing saline solution at a concentration between from about 0.1 to about 10mM. The buffer is used to maintain physiological pH. Any buffer or buffer system which is capable of maintaining physiological pH may be used.

Examples of acceptable buffers and buffer systems include sodium bicarbonate, disodium phosphate/sodium phosphate, and monobasic potassium phosphate/sodium hydroxide. Likewise, any antiseptic preservative which is capable of preserving the sterility of the nasal moisturizing saline solution may be used. Examples of acceptable preservatives include phenylcarbinol, benzalkonium chloride, and thimerosal.

### EXAMPLE 5

The nasal solution of example 4 was used to treat allergic rhinitis in a 50 year old male by the nasal inhalation use described above. The nasal solution of example 4 prevented both nasal irritation and "rebound" congestion. Rebound congestion occurs when the commercial product of example 4 is used by the subject for more than 3 days. Rebound congestion does not clear up with the continued use of the commercial product. The solution of example 4 was administered in accordance with its labeled directions as follows. (squeeze bottle) with head upright, insert nozzle in nostril. Spray quickly, firmly and sniff deeply. Adults and children 6 years of age and over, spray 2 or 3 times into each nostril. Repeat twice daily-morning and evening.

In a preferred embodiment, the pyruvate is present in the nasal moisturizing saline solution at a concentration of about 5mM, the buffer is sodium bicarbonate, and the preservative is phenylcarbinol.

In another preferred embodiment, the pyruvate is present in the nasal moisturizing saline solution at a concentration of about 2.5mM, the buffer is sodium bicarbonate, and the preservative is phenylcarbinol.

In another preferred embodiment, the pyruvate is present in the nasal moisturizing saline solution at a concentration of about 1.5mM, the buffer is sodium bicarbonate, and the preservative is phenylcarbinol.

The pyruvate-containing nasal solution may be applied to the mucous membranes of the nose by using nose drops or a nose spray. Before using nose drops or sprays, the patient should gently blow his/her nose if he/she can. For the administration of the nose drops, the patient should fill the dropper, tilt his/her head back, and place the prescribed number of drops into his/her nose. To prevent contamination of the rest of the solution, the dropper should not touch the nasal membranes. The patient should keep his/her head titled for five to ten seconds, and sniff gently two or three times.

When using a nasal spray, however, the patient should not tilt his/her head back. The sprayer should be inserted into the nose, but without touching the inner nasal membranes. The patient should sniff and squeeze the sprayer at the same time. The patient should not release his/her grip on the sprayer until it has been withdrawn from the nose in order to prevent nasal mucus and bacteria from entering the plastic bottle and contaminating its contents. After the patient has sprayed the prescribed number of times in one or both nostrils, he/she should gently sniff two or three times.

The following specific Examples are used to illustrate the use for the prevention and/or treatment of rhinitis, eosiophilis syndrome, sinusitis and related conditions associated with nasal congestion of the present invention.

Particular disease states to be treated are rhinitis and sinusitus .

Bronchial asthma has been defined as an inflammatory disorder of the airways. However upper airways are often forgotten either by the clinicians when they care for asthmatics or the designers of clinical trials. Nose and sinus are a part of the airways and their role appear to be important in the pathophysiology of asthma; the treatment of rhinitis, eosinophilia syndrome and sinusitis has to be included in the recommendations. The inflammatory mediator of the present invention may be used to treat all of these conditions when they appear separately or in consort.

As the most common allergic manifestation, rhinitis affects some 20% of the general population and remains on the rise, especially in industrialized nations, where nasal mucosae continue to be bombarded by an ever-expanding array of allergens and irritants. Though much allergic rhinitis is seasonal, perennial rhinitis is now more common, and its increased incidence outpaces that of seasonal rhinitis. Elucidation of the mechanisms involved in the immune response is essential in devising effective treatments.

Allergic rhinitis is an allergic reaction in which the body tries to repel foreign substances. This type of reaction is useful in combating foreign agents such as viruses and bacteria; but in people with allergies, the powerful response to relatively harmless substances (such as grass pollen) amounts to overkill.
1.Antigens are processed by antigen-presenting cells (APCs-including Langerhans cells) in the nasal mucosa, broken down into smaller amino acid chains that bind to MHC class II molecules which determine whether or not an immune response is mounted.
2.APCs migrate to the lymphoid tissues to present the antigens to Th0 lymphocytes from the thymus that subsequently differentiate to Th2 lymphocytes responsible for specific cytokine production and stimulation of IgE production by B cells.
3.These B cells bear IgM specific to sensitized antigen and, through heavy-chain switching, produce specific IgE that may remain tissue-bound on mast cells of basophils or be released into general circulation. Eventually they become more reactive to the specific antigens
   Differential Classification of Rhinitis:
   Allergic: Seasonal, Perennial
   Non-Allergic: Infectious; Viral, Viral Accompanied By Sinusitis
   Non-infectious; Vasomotor With Eosinophilia (NARES)
4.Degranulation of mast cells during the immediate allergic response and of basophils 4 to 6 hours later releases not only histamine, but tryptase, prostaglandins (PGD2 and PGF2), and bradykinin, which helps to explain why antihistamines are not the allergic "cure-all" that they were once regarded.
5.Peptidyl leukotrienes (LTC-4, LTD-4, and LTE-4) released by mast cells and eosinophils increase vascular permeability, potentiate airway hyperresponsiveness, and stimulate glandular exocytosis.
6.Histamine is now recognized to react with H1 receptors on nociceptive type C nerves of the mucosa and submucosa to stimulate release of substance P and other neurotransmitters, accounting for some measure of neurogenic inflammation.
7.The late-phase allergic response is characterized by the migration of inflammatory eosinophils, basophils, and neutrophils into affected tissues, attracted by cytokines released during the early phase.
8.Inflammatory cell migration is facilitated by a variety of complex interactions with adhesion molecules as well as the increased permeability of microvascular endothelial beds
9.Once the process is underway, eosinophils become a major player in the pathophysiology of allergic airway disease, with eosinophil cationic protein, eosinophil-derived neurotoxin, eosinophil peroxidase, and major basic protein binding to proteoglycans and hyaluronan of the basement membrane to cause epithelial desquamation and cellular disaggregation. (O'Hollaren M. Update in allergy and immunology. Annals of Internal Medicine. 1998;129:1036-1043; Harbus T, ed. Cost-effective treatment of rhinitis: a managed care perspective. The American Journal of Managed Care. September 1997;3(Suppl). Roundtable discussions.)

Almost every child or adult will experience one or more episodes of acute infectious rhinitis due to viruses in their lifetime. The most common types of rhinitis that are not due to acute viral infections include allergic rhinitis, nonallergic rhinitis of the vasomotor type and infectious rhinitis with sinusitis. Although much less common, other types of rhinitis should be recognized so that proper investigation and treatment can be given.

Many classification schemes for rhinitis exist, and all are valid and useful. Some classifications are based on the different pathophysiologies underlying the various types of rhinitis; others are based on their different clinical presentations. Another classification scheme is based on the presence or absence of nasal eosinophilia. Because the pathophysiology of all the types of rhinitis is not known and nasal cytology smears for the determination of nasal eosinophilia are not uniformly available to clinical practitioners, a simple classification scheme for rhinitis is presented below.

### Allergic rhinitis

Allergic rhinitis is a nasal inflammatory disorder initiated by an IgE-mediated hypersensitivity to foreign substances, i.e. allergens. To cause rhinitis, an airborne allergen must contact the respiratory mucosa. Increased amounts of allergen in the ambient air correlate well with rhinitis symptoms. Particles the size of pollens, some moulds and their larger fragments (2 to 60 µm) are deposited on the nasal mucosa. In addition, pollen antigens can be detected in particle-free fractions of air. These water-soluble antigens make contact with both upper and lower respiratory tract mucosa and lead to the formation of specific IgE antibody in susceptible hosts. Clinically, allergic rhinitis is manifested by nasal congestion, itching of eyes, nose and palate, rhinorrhea, and episodes of repetitive sneezing. Itching of the eyes, nose and palate is significantly more common in seasonal allergic rhinitis than in perennial rhinitis.

Many different cells and molecules account for the pathophysiology of allergic rhinitis. Among them, T cells and their secreted products play an important role.

Production of IgE antibodies requires two signals. The initial meeting of an allergen and the immune system yields no symptoms; rather, it may prepare the body to react promptly to future encounters. The sensitization process begins when macrophages degrade the allergen and display the resulting fragments to the CD4+ T lymphocytes. This results in T-cell activation events that lead to the synthesis of new proteins that enable T helper (Th) cells to deliver signals to B cells. To produce IgE antibodies, resting B cells require an initial signal delivered by physical interaction with activated CD4+ T lymphocytes. Such contact between B and T cells may consist of recognition by the T-cell antigen receptor (TCR)-CD3 complex of allergen peptides in combination with major histocompatibility complex (MHC) class II determinants on B cells. These interactions may also involve different molecules on the surface of lymphocytes, including CD on B cells and the CD ligand on activated T cells. Another important signal required for synthesis of IgE is provided by the interleukin, IL-4.

Production of IL-4 by CD4+ T lymphocytes. The unusually high levels of IgE that characterize atopic patients are partly explained by the nature of the T cells in these patients. Studies of T-cell clones have delineated two major subpopulations of CD4+ T cells, Th1 and Th2 cells, which secrete different cytokines following activation. Th1 cells secrete IL-2, interferon-y (IFN-y) and lymphotoxin, whereas Th2 cells secrete IL-4, IL-5, IL-6 and IL-10. Other lymphokines, such as IL-3 and granulocyte-macrophage colony-stimulating factor (GM-CSF), are secreted by both cell types.

Because Th2 cells produce IL-4, it has been postulated that these cells are more represented in atopic patients and are involved in the elevated IgE production. It has been reported that the majority of allergen-specific T-cell clones derived from atopic donors bear the Th2 phenotype. Interestingly, Th2 cells were found in the skin of atopic donors following allergen-induced late-phase cutaneous reactions and they were predominant in the bronchoalveolar lavage of subjects with atopic asthma.

An important feature of Th1 and Th2 cells is the ability of one subset to regulate the activities of the other, as indicated by the inhibitory effects of IFN-y on IL-4-induced B-cell activation or those of IL-4 on IL-2-induced T- and B-lymphocyte proliferation. It has also been demonstrated that IFN-y inhibits the proliferation of Th2 cells, whereas IL-10 inhibits cytokine production by Th1 cells. The mechanisms controlling the development of Th1 and Th2 cells are poorly understood, but likely depend on antigen structure, antigen exposition, HLA phenotype, antigen presenting cells (APC) and locally active steroid hormones.

Once produced, IgE antibodies attach to the receptors on mast cells in tissue and basophils circulating in blood. In later encounters between allergen and the body, allergen molecules promptly bind to IgE on mast cells. When an allergen molecule connects two IgE antibodies on the cell surface, it draws the attached IgE receptors together, thereby directly or indirectly activating various enzymes in the cell membrane. Cascade reactions involving many cellular enzymes and an influx of calcium ions induce chemical-laden granules to release their contents. The release of histamine and other preformed mediators causes the immediate allergic symptom of nasal obstruction due to vascular congestion and leakage with subsequent mucosal edema, in addition to the increased glandular secretions from submucosal glands. These same mediators also cause the immediate symptoms of pruritus and sneezing due to neural stimulation. The cascade reactions promote the synthesis of other inflammatory substances that can be identified in nasal secretions, including the leukotrienes C4, D4 and E4, prostaglandins and extracellularly derived kinins. These mediators are present in the early phase of the allergic response.

Slowly eluted and newly synthesized mediators support reactions not apparent until 4 to 24 hours after mast-cell activation. This late-phase reaction in the nose produces allergic symptoms associated with infiltration of inflammatory cells. The late-phase reaction is associated with the reappearance of some, but not all, inflammatory mediators, an increased responsiveness to the allergen and hyperresponsiveness to irritants.

Complex neural mechanisms also contribute to the symptoms of rhinitis because mast-cell mediators affect the irritant receptor endings and cause sneezing or nasal congestion. In addition, the nonadrenergic, noncholinergic (NANC) system can cause direct effects.

Other cells besides mast cells are also important in chronic allergic inflammation. CD4+ lymphocytes may also play a central role in the differentiation, selective recruitment and accumulation of inflammatory cells in target organs, as well as the activation and persistence of cells directly involved in tissue inflammation. At the level of the respiratory mucosa, these cells can be activated by allergen presented by antigen presenting cells. T cells possess long-term immunologic memory and can rapidly proliferate, recirculate and be easily recruited to target tissues. Through the release of different combinations of cytokines, they represent a common factor linking IgE production, eosinophils, mast cells, basophils and macrophages.

Hematopoietic growth factors, peptide growth factors, interleukins, interferons and histamine-releasing factors (HRFs) are among the cytokines that probably play important roles in allergic inflammation. The hematopoietic differentiation of allergic effector cells, including basophils, mast cells and eosinophils, is mediated by growth factors such as IL-339 and GM-CSF. IL-3 stimulates the basophil lineage from a common basophil-eosinophil progenitor. GM-CSF probably synergizes both basophil and eosinophil differentiation. IL-5, produced by Th2 cells, is a specific eosinophil growth and differentiation factor.

Histamine-releasing factors have been shown to modulate the secretory response of mast cells and basophils. Histamine-releasing factors, defined as cell products that cause basophil degranulation and histamine release, can be produced by a wide variety of cells including neutrophils, platelets, alveolar macrophages and mononuclear cells.

Nonallergic rhinitis comprises a heterogenous group of disorders. Although its prevalence is not known precisely, nonallergic rhinitis constitutes a significant proportion of patients seen for rhinitis assessment and management. Although different terms have been used to categorize and describe different types of nonallergic rhinitis, patients may present with variable nasal symptoms and may also complain predominantly of one symptom, such as rhinorrhea, compared with another, such as nasal congestion. For most types of nonallergic rhinitis, the exact pathophysiology is unknown.

Vasomotor rhinitis constitutes a symptom complex of sneezing and watery rhinorrhea with or without nasal congestion with symptoms that can develop rapidly but also resolve quickly. Although some authors believe that vasomotor rhinitis is a "pure" syndrome consisting of watery rhinorrhea and sneezing without nasal congestion, clinical practitioners who see patients with rhinitis commonly see large numbers of patients with the combination of sneezing, rhinorrhea and nasal congestion in whom an allergic or infectious cause cannot be found and who do not fit into any clearly defined rhinitis classification. It is useful to categorize these patients for clinical purposes as having nonallergic vasomotor rhinitis, although this classification continues to be far from satisfactory in defining an etiology for their symptoms. Triggers, such as bright lights, odours associated with such substances as detergents or cooked foods, cold air, exercise, eating, emotional upset and even sexual activity, can provoke the nasal symptoms. IgE-mediated mechanisms do not play a role in vasomotor rhinitis, and the exact pathophysiologic mechanisms underlying the condition are unknown. Cholinergic hyperresponsiveness has been shown in patients with perennial rhinitis and excessive nasal secretions, and topical anticholinergic medications have been shown to reduce the watery secretions in patients with vasomotor rhinitis. Inflammatory cells are absent from the mucosal secretions.

Infectious rhinitis and sinusitis: Although the common cold is the most frequent presentation of acute infectious rhinitis, other infectious agents may reside in the nose and be transmitted to the lower respiratory tract. Bacterial infections may develop in the nose. Except perhaps for localized staphylococcal vestibulitis or those infections associated with atrophic rhinitis, bacterial infections invariably involve the nasal sinuses as well as the nose.

Chronic sinusitis is an important cause of secondary infectious rhinitis, and chronic infectious rhinitis is more aptly termed rhinosinusitis because ethmoid air cells are commonly infected. Patients with chronic infectious rhinitis complain of nasal congestion, halitosis, purulent rhinorrhea and cough due to postnasal drip. In these patients, the secretions of the infected sinus invade the nose, and they contain neutrophils rather than eosinophils. Sinusitis is demonstrable in plain films or by computed tomography (CT) scanning. The pathophysiology of rhinosinusitis includes the generation of chemotactic factors by complement activation through the associated interaction of bacterial metabolites with epithelium-bound macrophages, which can further amplify the inflammatory response. In patients with immune deficiency, refractory chronic bacterial infectious rhinosinusitis may occur, and consideration should also be given to nasal fungal and mycobacterial infections. In infectious rhinitis, the nasal infection may be limited to the nasal passages, but in other systemic viral infections, such as varicella, the nasal infection is only part of the systemic symptoms.

Nonallergic rhinitis with eosinophilia syndrome (NARES): Nonallergic rhinitis with eosinophilia syndrome (NARES) may be manifested by significant pruritus, sneezing and watery rhinorrhea, and symptoms are characteristically perennial with paroxysmal exacerbations. Polypoid changes may occur in the nasal mucosa. Although symptoms in patients with NARES may be identical to those in patients with allergic rhinitis and nasal secretions contain abundant eosinophils, there is no evidence of IgE-mediated hypersensitivity. The pathophysiologic mechanisms of this syndrome are unknown.

Rhinitis medicamentosa and rhinitis due to systemic medications: Rhinitis medicamentosa (medication-induced rhinitis) may be seen in patients who chronically use nasal decongestant sprays or occasionally other over-the-counter intranasal preparations. Patients develop nasal congestion due to nasal mucosal vascular dilatation and edema. In other patients, rhinitis may be due to the use of systemic medications and antihypertensive drugs (e.g., beta-blockers) are most frequently implicated. The recreational usage of cocaine intranasally can be associated with nasal mucosal inflammation and congestion, as well as nasal septal perforation.

Rhinitis due to hormones: In patients with rhinitis associated with pregnancy, severe nasal congestion, sneezing and profuse rhinorrhea are often seen during the second and third trimesters. These symptoms usually subside within several weeks after delivery and the return to a normal estrogen cycle. The intranasal vascular engorgement and mucosal hypersecretion during pregnancy are thought to be hormone related, although why this condition affects some, but not all, pregnant women is unknown.

Atrophic rhinitis: The nasal mucosa must be moist for the patient to perceive that nasal air flow is normal and not obstructed. Although more commonly associated with a prior severe nasal infection, trauma or surgery, atrophic rhinitis can be a rare primary condition. Patients with atrophic rhinitis frequently give a history of severe nasal obstruction, yet physical examination reveals that the nasal passages are patent with no apparent obstruction to air flow. The mucosa looks dry, and nasal crusting may be present. In patients with primary atrophic rhinitis, the crusting can become thick and malodorous, detectable by both patient and the physician on nasal examination. Vigorous nasal lubrication including the use of topical saline, lubricant gels and sprays, as well as antibiotic treatment of any associated bacterial infection may provide symptom relief.

Rhinitis associated with other diseases and anatomic abnormalities: Rhinitis may rarely be a manifestation of an associated systemic disease. Examples of such diseases with superimposed infections associated with rhinitis include cystic fibrosis, immotile cilia syndrome and immune deficiency including HIV infection. Systemic diseases with rhinitis, but without associated infections, include severe myxedema and autoimmune granulomatous disorders. Autoimmune granulomatous disorders, such as Wegener's granulomatosis and sarcoidosis, may affect both the nose and paranasal sinuses and present as rhinitis and chronic sinusitis. Diagnosis is challenging and is usually made by examining the pattern of the multisystem involvement that accompanies the nasal and sinus symptoms. The diagnosis is confirmed by nasal mucosal biopsy showing granulomatous changes and by the presence of antineutrophil cytoplasmic antibody (ANCA) in the blood of the patient with Wegener's granulomatosis.

Patients presenting with rhinitis should also be assessed for congenital and acquired anatomic causes of nasal obstruction. Reduced air flow through the nasal passages in infants may be due to congenital choanal atresia. The most common acquired anatomic cause of nasal obstruction in infants and children is adenoidal hypertrophy. Deviated nasal septum, nasal polyps or an impacted foreign object may also cause obstruction. Neoplastic disorders that cause obstruction (such as epithelial carcinomas, lymphoma, sarcoma and angiofibroma) may occur rarely. The term rhinitis as used herein, unless otherwise indicated, refers to all forms of rhinitis.

The inflammatory mediator of the present invention may be administered prior to, after and/or with other therapeutic agents. Typical therapeutic agents are antibacterials, antivirals, antifungals, antihistamines, proteins, enzymes, hormones, nonsteroidal anti-inflammatories, cytokines, steroids, insulin, vitamins and the like.

When the therapeutic agent is insulin, the insulin is present in an amount from about 25 to 500 units per ml.; preferably the insulin is present from about 25 to 200 units per ml.; more preferably from about 50 to 150 units per ml; and most preferably from about 75 to about 150 units per ml.

## Claims

1. An inflammatory mediator which is an antioxidant, wherein the inflammatory mediator is a pyruvate precursor, pyruvate, or a mixture thereof, wherein the pyruvate precursor is selected from pyruvyl-glycine, pyruvyl-alanine, pyruvyl-leucine, pyruvyl-cysteine, pyruvyl-valine, pyruvyl-isoleucine, pyruvyl-phenylalanine, pyruvamide, dihydroxyacetone and salts of pyruvic acid, for use in treating rhinitis, eosinophilia syndrome and/or sinusitis.

2. The inflammatory mediator of claim 1 for use in treating rhinitis, eosinophilia syndrome and/or sinusitis, wherein the treatment comprises contacting cells of the mucous membranes of nasal and sinus passages of a mammalian subject with an amount of the inflammatory mediator which is capable of reducing an undesired inflammatory response in said cells.

3. The inflammatory mediator of claim 1 or claim 2, wherein the inflammatory mediator is formulated into nasal drops.

4. The inflammatory mediator of claim 1 or claim 2, wherein the inflammatory mediator is formulated into a nasal ointment.

5. The inflammatory mediator of claim 3 or claim 4, wherein the inflammatory mediator is formulated in a concentration of 0.1 mM to 10.0 mM.

6. The inflammatory mediator of any one of claims 2 to 5, wherein the inflammatory response being reduced is at least one of the following: oxygen radical production, hydrogen peroxide production, cytokine and protease production, prostiglandin production, erythema, histamine and interleukin production.

7. The inflammatory mediator of any one of claims 1 to 6 which is pyruvate.

8. The inflammatory mediator of claim 7, wherein the pyruvate is selected from pyruvic acid, lithium pyruvate, sodium pyruvate, potassium pyruvate, magnesium pyruvate, calcium pyruvate, zinc pyruvate, manganese pyruvate; and mixtures thereof.

9. The inflammatory mediator of any one of claims 1 to 8, wherein the treatment further comprises contacting cells of the mucous membranes of nasal and sinus passages with a therapeutic agent.

10. The inflammatory mediator of claim 9, wherein the therapeutic agent is administered prior to the inflammatory mediator, or concomitantly with administration of the inflammatory mediator, or after administration of the inflammatory mediator.

11. The inflammatory mediator of claim 9 or claim 10, wherein the therapeutic agent is one or more agents selected from antibacterials, antivirals, antifungals, antihistamines, proteins, enzymes, hormones, nonsteroidal anti-inflammatories, cytokines, insulin, vitamins and steroids.

12. The inflammatory mediator of claim 11, wherein the therapeutic agent is oxymetazoline.

13. A nasal solution, comprising:
a) water,
b) sodium chloride, 0.65% by weight,
c) pyruvate, at least 0.1 mM,
d) buffer, and optionally
e) a preservative,
wherein the nasal solution is a moisturizing saline solution and is buffered and made isotonic.

14. The nasal solution of claim 13, wherein the pyruvate is present in the solution at a concentration between from about 0.1 mM to about 10 mM.

15. The nasal solution of claim 13 or claim 14, wherein the buffer is selected from sodium bicarbonate, disodium phosphate/sodium phosphate, and monobasic potassium phosphate/sodium hydroxide.

16. The nasal solution of any one of claims 13 to 15, wherein the preservative is selected from phenylcarbinol, benzalkonium chloride and thimerosal.

17. The nasal solution of any one of claims 13 to 16, further comprising a therapeutic agent wherein the therapeutic agent is one or more agents selected from antibacterials, antivirals, antifungals, antihistamines, proteins, enzymes, hormones, nonsteroidal anti-inflammatories, cytokines, insulin, vitamins and steroids.

18. The nasal solution of claim 17, wherein the therapeutic agent is oxymetazoline.

19. A nasal solution as claimed in any one of claims 13 to 18 for the prevention and/or treatment of rhinitis, eosinophilia syndrome, sinusitis and related conditions associated with nasal congestion.

20. A nasal solution as claimed in claim 19 for the prevention and/or treatment of rhinitis, eosinophilia syndrome, sinusitis and related conditions associated with nasal congestion, wherein the prevention and/or treatment comprises administering the nasal solution to the nostrils of a patient in need thereof.

21. Use of an inflammatory mediator which is an antioxidant, wherein the inflammatory mediator is pyruvate precursor, pyruvate, or a mixture thereof, wherein the pyruvate precursor is selected from pyruvyl-glycine, pyruvyl-alanine, pyruvyl-leucine, pyruvyl-cysteine, pyruvyl-valine, pyruvyl-isoleucine, pyruvyl-phenylalanine, pyruvamide, dihydroxyacetone and salts of pyruvic acid, in the manufacture of a medicament for treating rhinitis, eosinophilia syndrome and/or sinusitis.

22. The use as claimed in claim 21, wherein the treatment comprises contacting cells of the mucous membranes of nasal and sinus passages of a mammalian subject with an amount of the medicament which is capable of reducing an undesired inflammatory response in said cells.

23. Use of a nasal solution as claimed in any one of claims 13 to 18 in the manufacture of a medicament for the prevention and/or treatment of rhinitis, eosinophilia syndrome, sinusitis and related conditions associated with nasal congestion.

24. The use as claimed in claim 23, wherein the prevention and/or treatment comprises administering the medicament to the nostrils of a patient in need thereof.

## Patentansprüche

1. Entzündungsmediator, der ein Antioxidans ist, wobei der Entzündungsmediator ein Pyruvatvorläufer, ein Pyruvat oder ein Gemisch davon ist, wobei der Pyruvatvorläufer aus Pyruvyl-Glycin, Pyruvyl-Alanin, Pyruvyl-Leucin, Pyruvyl-Cystein, Pyruvyl-Valin, Pyruvyl-Isoleucin, Pyruvyl-Phenylalanin, Pyruvamid, Dihydroxyaceton und Salzen von Brenztraubensäure ausgewählt ist, zur Verwendung bei der Behandlung von Rhinitis, Eosinophilie-Syndrom und/oder Sinusitis.

2. Entzündungsmediator nach Anspruch 1 zur Verwendung bei der Behandlung von Rhinitis, Eosinophilie-Syndrom und/oder Sinusitis, wobei die Behandlung das Kontaktieren von Zellen der Schleimhäute von Nasen- und Nasennebenhöhlenwegen eines Säugetiers mit einer Menge des Entzündungsmediators umfasst, die in der Lage ist, eine unerwünschte Entzündungsreaktion in den Zellen zu verringern.

3. Entzündungsmediator nach Anspruch 1 oder Anspruch 2, wobei der Entzündungsmediator in Nasentropfen formuliert ist.

4. Entzündungsmediator nach Anspruch 1 oder Anspruch 2, wobei der Entzündungsmediator in eine Nasensalbe formuliert ist.

5. Entzündungsmediator nach Anspruch 3 oder Anspruch 4, wobei der Entzündungsmediator in einer Konzentration von 0,1 mM bis 10,0 mM formuliert ist.

6. Entzündungsmediator nach einem der Ansprüche 2 bis 5, wobei die Entzündungsreaktion, die verringert wird, mindestens eine der folgenden ist: Sauerstoffradikal-Erzeugung, Wasserstoffperoxid-Erzeugung, Zytokin- und Proteaseerzeugung, Prostaglandin-Erzeugung, Erythem-, Histamin- und Interleukin-Erzeugung.

7. Entzündungsmediator nach einem der Ansprüche 1 bis 6, der ein Pyruvat ist.

8. Entzündungsmediator nach Anspruch 7, wobei das Pyruvat aus Brenztraubensäure, Lithiumpyruvat, Natriumpyruvat, Kaliumpyruvat, Magnesiumpyruvat, Kalziumpyruvat, Zinkpyruvat, Manganpyruvat und Gemischen davon ausgewählt ist.

9. Entzündungsmediator nach einem der Ansprüche 1 bis 8, wobei die Behandlung weiterhin das Kontaktieren von Zellen der Schleimhäute von Nasen- und Nasennebenhöhlenwegen mit einem therapeutischen Mittel umfasst.

10. Entzündungsmediator nach Anspruch 9, wobei das therapeutische Mittel vor dem Entzündungsmediator oder gleichzeitig mit der Verabreichung des Entzündungsmediators oder nach der Verabreichung des Entzündungsmediators verabreicht wird.

11. Entzündungsmediator nach Anspruch 9 oder Anspruch 10, wobei das therapeutische Mittel ein oder mehrere Mittel ist, die aus antibakteriellen Mitteln, antiviralen Mitteln, fungiziden Mitteln, Antihistaminen, Proteinen, Enzymen, Hormonen, nicht steroidalen entzündungshemmenden Mitteln, Zytokinen, Insulin, Vitaminen und Steroiden ausgewählt sind.

12. Entzündungsmediator nach Anspruch 11, wobei das therapeutische Mittel Oxymetazolin ist.

13. Nasenlösung, die enthält:
a) Wasser,
b) Natriumchlorid, 0,65 Gewichts-%,
c) Pyruvat, mindestens 0,1 mM,
d) Puffer und wahlweise
e) ein Konservierungsmittel,
wobei die Nasenlösung eine befeuchtende Salzlösung ist und gepuffert und isotonisch gemacht ist.

14. Nasenlösung nach Anspruch 13, wobei das Pyruvat in der Lösung mit einer Konzentration zwischen etwa 0,1 mM und etwa 10 mM vorliegt.

15. Nasenlösung nach Anspruch 13 oder Anspruch 14, wobei der Puffer aus Natriumbicarbonat, Dinatriumphosphat/Natriumphosphat und einbasischem Kaliumphosphat/Natriumhydroxid ausgewählt ist.

16. Nasenlösung nach einem der Ansprüche 13 bis 15, wobei das Konservierungsmittel aus Phenylcarbinol, Benzalkoniumchlorid und Thimerosal ausgewählt ist.

17. Nasenlösung nach einem der Ansprüche 13 bis 16, die weiterhin ein therapeutisches Mittel aufweist, wobei das therapeutische Mittel ein oder mehrere Mittel ist, die aus antibakteriellen Mitteln, antiviralen Mitteln, fungiziden Mitteln, Antihistaminen, Proteinen, Enzymen, Hormonen, nicht steroidalen entzündungshemmenden Mitteln, Zytokinen, Insulin, Vitaminen und Steroiden ausgewählt sind.

18. Nasenlösung nach Anspruch 17, wobei das therapeutische Mittel Oxymetazolin ist.

19. Nasenlösung nach einem der Ansprüche 13 bis 18 für die Verhinderung und/oder Behandlung von Rhinitis, Eosinophilie-Syndrom, Sinusitis und verwandten Zuständen, die mit einer verstopften Nase verbunden sind.

20. Nasenlösung nach Anspruch 19 für die Verhinderung und/oder Behandlung von Rhinitis, Eosinophilie-Syndrom, Sinusitis und verwandten Zuständen, die mit einer verstopften Nase verbunden sind, wobei die Verhinderung und/oder Behandlung das Verabreichen der Nasenlösung an die Nasenlöcher eines Patienten mit Bedarf daran umfasst.

21. Verwendung eines Entzündungsmediators, der ein Antioxidans ist, wobei der Entzündungsmediator ein Pyruvatvorläufer, ein Pyruvat oder ein Gemisch davon ist, wobei der Pyruvatvorläufer aus Pyruvyl-Glycin, Pyruvyl-Alanin, Pyruvyl-Leucin, Pyruvyl-Cystein, Pyruvyl-Valin, Pyruvyl-Isoleucin, Pyruvyl-Phenylalanin, Pyruvamid, Dihydroxyaceton und Salzen von Brenztraubensäure ausgewählt ist, bei der Herstellung eines Medikaments für die Behandlung von Rhinitis, Eosinophilie-Syndrom und/oder Sinusitis.

22. Verwendung nach Anspruch 21, wobei die Behandlung das Kontaktieren von Zellen der Schleimhäute von Nasen- und Nasennebenhöhlenwegen eines Säugetiers mit einer Menge des Medikaments umfasst, die in der Lage ist, eine unerwünschte Entzündungsreaktion in den Zellen zu verringern.

23. Verwendung einer Nasenlösung nach einem der Ansprüche 13 bis 18 bei der Herstellung eines Medikaments für die Verhinderung und/oder Behandlung von Rhinitis, Eosinophilie-Syndrom, Sinusitis und verwandten Zuständen, die mit einer verstopften Nase verbunden sind.

24. Verwendung nach Anspruch 23, wobei die Verhinderung und/oder Behandlung das Verabreichen des Medikaments an die Nasenlöcher eines Patienten mit Bedarf daran umfasst.

## Revendications

1. Médiateur inflammatoire qui est un antioxydant, dans lequel le médiateur anti-inflammatoire est un précurseur du pyruvate, le pyruvate ou un mélange de ceux-ci, dans lequel le précurseur du pyruvate est choisi parmi la pyruvyl-glycine,la pyruvyl-alanine, la pyruvyl-leucine, la pyruvyl-cistéine, la pyruvyl-valine, la pyruvyl-isoleucine, la pyruvyl-phénylalanine, le pyruvamide, la dihydroxyacétone et des sels d'acide pyruvique, pour une utilisation dans le traitement de la rhinite, du syndrome d'eosinophilie et/ou de la sinusite.

2. Médiateur inflammatoire selon la revendication 1 pour une utilisation dans le traitement de la rhinite, du syndrome d'eosinophilie et/ou de la sinusite, dans lequel le traitement comporte l'étape consistant à mettre en contact des cellules des muqueuses de voies nasales et sinusales d'un sujet mammifère avec une quantité du médiateur inflammatoire qui est capable de réduire une réponse inflammatoire non voulue dans lesdites cellules.

3. Médiateur inflammatoire selon la revendication 1 ou la revendication 2, dans lequel le médiateur inflammatoire est formulé en gouttes nasales.

4. Médiateur inflammatoire selon la revendication 1 ou la revendication 2, dans lequel le médiateur inflammatoire est formulé en onguent nasal.

5. Médiateur inflammatoire selon la revendication 3 ou la revendication 4, dans lequel le médiateur inflammatoire est formulé dans une concentration de 0,1 mM à 10,0 mM.

6. Médiateur inflammatoire selon l'une quelconque des revendications 2 à 5, dans lequel la réponse inflammatoire réduite est au moins une parmi les suivantes : production de radicaux oxygène, production de peroxyde d'hydrogène, production de cytokines et de protéases, production de prostiglandine, production d'érythème, d'histamine et d'interleukines.

7. Médiateur inflammatoire selon l'une quelconque des revendications 1 à 6, lequel est le pyruvate.

8. Médiateur inflammatoire selon la revendication 7, dans lequel le pyruvate est choisi parmi l'acide pyruvique, le pyruvate de lithium, le pyruvate de sodium, le pyruvate de potassium, le pyruvate de magnésium, le pyruvate de calcium, le pyruvate de zinc, le pyruvate de manganèse, et des mélanges de ceux-ci.

9. Médiateur inflammatoire selon l'une quelconque des revendications 1 à 8, dans lequel le traitement comporte en outre l'étape consistant à mettre en contact des cellules des muqueuses de voies nasales et sinusales avec un agent thérapeutique.

10. Médiateur inflammatoire selon la revendication 9, dans lequel l'agent thérapeutique est administré avant le médiateur inflammatoire, ou de manière concomitante avec l'administration du médiateur inflammatoire, ou après l'administration du médiateur inflammatoire.

11. Médiateur inflammatoire selon la revendication 9 ou la revendication 10, dans lequel l'agent thérapeutique est un ou plusieurs agents choisis parmi des antibactériens, des antiviraux, des antifongiques, des antihistaminiques, des protéines, des enzymes, des hormones, des anti-inflammatoires non stéroïdiens, des cytokines, de l'insuline, des vitamines et des stéroïdes.

12. Médiateur inflammatoire selon la revendication 11, dans lequel l'agent thérapeutique est l'oxymétazoline.

13. Solution nasale, comportant :
a) de l'eau
b) du chlorure de sodium, 0,65 % en poids,
c) du pyruvate, au moins 0,1 mM,
d) un tampon, et facultativement
e) un agent de conservation,
dans laquelle la solution nasale est une solution saline hydratante et est tamponnée et rendue isotonique.

14. Solution nasale selon la revendication 13, dans laquelle le pyruvate est présent dans la solution à une concentration comprise entre environ 0,1 mM et environ 10 mM.

15. Solution nasale selon la revendication 13 ou la revendication 14, dans laquelle le tampon est choisi parmi le bicarbonate de sodium, le phosphate de dissodium/le phosphate de sodium, et le phosphate de potassium/l'hydroxyde de sodium monobasique.

16. Solution nasale selon l'une quelconque des revendications 13 à 15, dans laquelle l'agent de conservation est choisi parmi le phénylcarbinol, le chlorure de benzalconium et le thimérosal.

17. Solution nasale selon l'une quelconque des revendications 13 à 16, comportant en outre un agent thérapeutique, dans laquelle l'agent thérapeutique est un ou plusieurs agents choisis parmi des antibactériens, des antiviraux, des antifongiques, des antihistaminiques, des protéines, des enzymes, des hormones, des anti-inflammatoires non stéroïdiens, des cytokines, de l'insuline, des vitamines et des stéroïdes.

18. Solution nasale selon la revendication 17, dans laquelle l'agent thérapeutique est l'oxymétazoline.

19. Solution nasale telle que revendiquée dans l'une quelconque des revendications 13 à 18 pour la prévention et/ou le traitement de la rhinite, du syndrome d'eosinophilie, de la sinusite et d'états apparentés associés à la congestion nasale.

20. Solution nasale telle que revendiquée dans la revendication 19 pour la prévention et/ou le traitement de la rhinite, du syndrome d'eosinophilie, de la sinusite et d'états apparentés associés à la congestion nasale, dans laquelle la prévention et/ou le traitement comporte l'administration de la solution nasale aux narines d'un patient ayant besoin de celle-ci.

21. Utilisation d'un médiateur inflammatoire qui est un antioxydant, dans laquelle le médiateur inflammatoire est un précurseur du pyruvate, le pyruvate ou un mélange de ceux-ci, dans laquelle le précurseur du pyruvate est choisi parmi la pyruvyl-glycine, la pyruvyl-alanine, la pyruvyl-leucine, la pyruvyl-cistéine, la pyruvyl-valine, la pyruvyl-isoleucine, la pyruvyl-phénylalanine, le pyruvamide, la dihydroxyacétone et des sels d'acide pyruvique, dans la fabrication d'un médicament destiné au traitement de la rhinite, du syndrome d'eosinophilie et/ou de la sinusite.

22. Utilisation telle que revendiquée dans la revendication 21, dans laquelle le traitement comporte l'étape consistant à mettre en contact des cellules des muqueuses de voies nasales et sinusales d'un sujet mammifère avec une quantité du médicament qui est capable de réduire une réponse inflammatoire non voulue dans lesdites cellules.

23. Utilisation d'une solution nasale telle que revendiquée dans l'une quelconque des revendications 13 à 18 dans la fabrication d'un médicament destiné à la prévention et/ou au traitement de la rhinite, du syndrome d'eosinophilie, de la sinusite et d'états apparentés associés à la congestion nasale.

24. Utilisation telle que revendiquée dans la revendication 23, dans laquelle la prévention et/ou le traitement comporte l'administration du médicament aux narines d'un patient ayant besoin de celui-ci.
